# DEMANDE DE BREVET EUROPEEN

(11) **EP 2 204 166 A2**
(43) Date de publication de la demande: **07.07.2010**
(21) Numéro de dépôt: 09178457.9
(22) Date de dépôt: 09.12.2009
(51) Int. Cl.: A61K 8/73, A61K 8/97, A61Q 3/02, A61K 8/36, A61K 8/92

(54) **Vernis à ongles comprenant une huile siccative, un polymère filmogène et un sel métallique**

(30) Priorité: 17.12.2008 FR 0858697; 23.12.2008 FR 0859035; 18.02.2009 US 153319 P; 21.01.2009 US 202023 P
(71) Demandeur: L'Oréal, 75008 Paris (FR)
(72) Inventeur: Kergosien, Guillaume, 92370, Chaville (FR); Samain, Henri, 91570, Bievres (FR)
(74) Mandataire: Le Coupanec, Pascale A.M.P.

(57) **Abrégé**

La présente invention a pour objet un vernis à ongles, **caractérisé en ce qu**'il comprend au moins une huile siccative, au moins un polymère filmogène et au moins un sel métallique.

La présente invention a également pour objet un procédé cosmétique de maquillage et/ou de soin non thérapeutique des ongles comprenant l'application sur les ongles d'au moins une couche dudit vernis à ongles.

## Description

La présente invention a pour objet un vernis à ongles comprenant au moins une huile siccative, au moins un polymère filmogène et au moins un sel métallique.

Cette composition de vernis à ongles peut être appliquée sur les ongles d'êtres humains ou bien encore sur des faux ongles.

La présente invention se rapporte en outre au procédé de maquillage et/ou de soin non thérapeutique des ongles correspondant.

La composition de vernis à ongles, colorée ou transparente, peut être employée comme base pour vernis ou « base-coat » en terminologie anglo-saxonne, comme produit de maquillage des ongles, comme composition de finition, encore appelée « top-coat », à appliquer sur le produit de maquillage des ongles ou bien encore comme produit de soin cosmétique des ongles.

Il est courant d'utiliser, dans les compositions de vernis à ongles, des agents filmogènes pour obtenir, après dépôt sur l'ongle, un film résistant présentant une bonne tenue. Actuellement, la nitrocellulose reste encore le filmogène le plus couramment utilisé dans les vernis à ongles présentant des propriétés de tenue optimisées.

Cependant, ces vernis à ongles comprenant de la nitrocellulose présentent l'inconvénient de ne pas être stables à la chaleur et au stockage.

Il en résulte notamment un jaunissement de ces compositions au cours du temps.

Par ailleurs, l'introduction de quantités importantes de nitrocellulose conditionne la composition de la phase solvant organique du vernis à ongle qui doit alors être compatible avec ce filmogène.

La présente invention a précisément pour but de fournir une composition de vernis à ongles comprenant une teneur réduite en nitrocellulose, voire exempte de nitrocellulose, présentant une bonne stabilité dans le temps, et permettant la formation d'un film homogène, brillant, et/ou non cassant.

Les inventeurs ont ainsi découvert qu'il était possible d'obtenir une telle composition comprenant moins de 5 % en poids en matière sèche de nitrocellulose par rapport au poids total de la composition, voire exempt de nitrocellulose, en utilisant au moins une huile siccative, au moins un polymère filmogène et au moins un sel métallique.

Ainsi, selon un de ses aspects, la présente invention a pour objet un vernis à ongles comprenant au moins une huile siccative, au moins un polymère filmogène et au moins un sel métallique.

Un tel vernis à ongles permet notamment une bonne stabilité dans le temps et d'obtenir un film solide, brillant, et/ou non cassant.

Selon un autre aspect, l'invention a encore pour objet un procédé cosmétique de maquillage et/ou de soin non thérapeutique des ongles comprenant l'application sur les ongles d'au moins une couche d'un vernis à ongles tel que défini précédemment.

Selon encore un autre de ses aspects, l'invention a encore pour objet l'utilisation d'un vernis à ongles tel que défini précédemment pour obtenir, après dépôt sur l'ongle, un film homogène, brillant et non cassant.

Le vernis à ongles selon l'invention comprend un milieu cosmétiquement acceptable, c'est-à-dire un milieu non toxique et susceptible d'être appliqué sur les matières kératiniques d'êtres humains, en particulier les ongles.

La présente invention concerne encore un support synthétique maquillé comprenant un maquillage obtenu par le procédé de l'invention.

Le vernis à ongles selon la présente invention peut prendre différentes formes soit une forme classique de vernis à ongles, c'est-à-dire qu'il présente une texture liquide ou gélifiée soit une forme d'article s'apposant et d'ajustant sur les ongles, comme exposé ci-après.

Selon un de ses aspects, le vernis à ongles selon l'invention présente une texture liquide ou gélifiée ; il diffère en particulier d'un patch ou article souple comprenant au moins une couche de matériau polymérique et une couche d'adhésif, destiné à être collé et ajusté grâce à ses propriétés de déformabilité, sur l'ongle.

Autrement dit, le vernis à ongles selon cet aspect de l'invention est typiquement appliqué sous forme de couches superposées à la surface des ongles ou des faux ongles à maquiller, par exemple à l'aide d'un pinceau.

Selon encore un autre de ses aspects, la présente invention a pour objet un article souple destiné à être appliqué sur les ongles et/ou faux ongles pour leur maquillage et/ou leur soin, comprenant au moins une couche polymérique, **caractérisé en ce que** ladite couche polymérique comprend au moins une huile siccative, au moins un polymère filmogène et au moins un sel métallique.

Ledit article souple est avantageusement introduit dans un conditionnement sensiblement étanche à l'air, le conditionnement étant tel que l'article s'y trouve conservé à l'abri de l'oxygène de l'air. Cet article souple est nommé pour des raisons de simplicité, dans la suite de la description, « article souple non totalement réticulé ».

Selon cet aspect de l'invention, l'article souple non totalement réticulé n'acquiert sa rigidité définitive qu'après application sur l'ongle et réticulation, par simple exposition à l'air ambiant.

La présente invention concerne également un procédé de maquillage et/ou de soin des ongles et/ou faux ongles comprenant au moins une étape consistant à appliquer sur un ongle naturel et/ou faux ongle, un article souple selon l'invention, qu'il soit considéré à l'état réticulé ou à l'état non totalement réticulé.

La présente invention concerne en outre un produit de maquillage et/ou de soin des ongles et/ou faux ongles comprenant, dans un conditionnement sensiblement étanche à l'air, au moins un article conforme à l'invention, le conditionnement étant tel que l'article s'y trouve conservé sous une forme partiellement sèche. Il peut être à l'état réticulé ou à l'état non totalement réticulé. Cet article souple est nommé pour des raisons de simplicité, dans la suite de la description, « article souple partiellement sec ».

Selon cet aspect de l'invention, l'article souple partiellement sec n'acquiert un aspect totalement sec, et donc sa forme définitive qu'après application sur l'ongle et séchage, par simple exposition à l'air ambiant.

La présente invention concerne enfin un procédé de maquillage et/ou de soin des ongles et/ou faux ongles comprenant au moins une étape consistant à appliquer sur un ongle naturel et/ou faux ongle, un article souple selon l'invention, qu'il soit considéré à l'état sec ou à l'état partiellement sec.

Selon un mode de réalisation, l'article souple conforme à la présente invention peut se présenter sous diverses formes telles qu'une étoile, un carré, un rond, etc.

Au sens de la présente invention, le terme « partiellement sec » entend qualifier le fait que l'article obtenu après formation du film n'est pas totalement exempt du solvant résiduel. En particulier, il possède une teneur en matière sèche supérieure à 80 %, plus particulièrement supérieure à 85 %, et inférieure à 95 % en poids par rapport à son poids total.

Dans une variante, l'article peut comprendre une superposition de couches polymériques.

Dans une autre variante, l'article peut comprendre une ou plusieurs couches adhésives en sus de la ou des couches polymériques.

Un article souple selon l'invention vise à proposer un mode de maquillage et/ou de soin des ongles ou des faux ongles présentant un pouvoir collant, dont la composition permette une souplesse et une capacité d'élongation suffisante au moment de l'application, et une rigidité satisfaisante après séchage.

De fait, il a été mis en évidence que l'utilisation, pour former une couche polymérique d'un article souple pour le maquillage et/ou le soin des ongles, d'une huile siccative, d'un polymère filmogène et d'un sel métallique pour former une couche polymérique d'un article souple pour le maquillage et/ou le soin des ongles et/ou faux ongles, permettait de former des films souples au moment de l'application, pouvant ainsi être étirés facilement lors de l'application sur les ongles, et devenir plus durs après plusieurs heures de séchage.

### I - COMPOSITION DE VERNIS A ONGLES SOUS FORME LIQUIDE OU GELIFIEE

### Huiles siccatives

Un vernis à ongles selon l'invention comprend au moins une huile siccative.

Par « huile siccative », on entend désigner une huile qui, lorsque étalée en couche mince puis exposée à l'air, se transforme en pellicule solide.

En particulier, on entend désigner dans le cadre de la présente invention par « huile siccative », les huiles, et de préférence les triglycérides, comportant des doubles liaisons conjuguées, de préférence comportant au moins deux doubles liaisons conjuguées, et de préférence comportant au moins trois doubles liaisons conjuguées.

Les huiles siccatives conformes à l'invention peuvent être d'origine naturelle.

De manière avantageuse, l'huile siccative peut être choisie parmi les huiles végétales siccatives telles que l'huile de lin, l'huile de bois de Chine (ou Canton), l'huile d'oïticica, l'huile de vernonia, l'huile d'oeillette, l'huile de grenade, l'huile de calendula ; les esters de ces huiles végétales ; les résines alkydes obtenues à partir de ces huiles végétales ; et leurs mélanges.

Les résides alkydes sont des polyesters comprenant des chaînes hydrocarbonées d'acides gras, obtenus notamment par polymérisation de polyols et de polyacides ou de leur anhydride correspondant, en présence d'acides gras. Ces acides gras sont présents, notamment sous la forme de triglycérides, dans la majorité des huiles naturelles, telles qu'en particulier les huiles citées précédemment.

L'huile siccative convenant à la mise en oeuvre de la présente invention peut être modifiée par réaction chimique.

En particulier, elle peut être raffinée et/ou partiellement polymérisée. A ce titre, on peut citer les huiles soufflées et les standolies, les huiles maléinisées, époxydées ou cuites.

Selon un mode de réalisation particulier, l'huile siccative de l'invention est distincte d'une huile époxydée.

Selon un mode de réalisation particulier de l'invention, l'huile siccative est une huile de lin raffinée.

Une huile peut être raffinée en particulier en trois étapes successives.

L'huile de lin raffinée selon l'invention peut ainsi résulter d'une étape de dégommage, pour obtenir en particulier une huile démucilaginée, suivie d'une étape de décoloration, en particulier pour la blanchir, puis d'une étape de neutralisation.

Selon un mode particulier de réalisation de l'invention, l'huile siccative de l'invention est une huile de lin modifiée selon au moins l'une des trois étapes mentionnées précédemment, autrement dit qui a subi soit une étape de dégommage, soit une étape de décoloration, soit une étape de neutralisation, soit une succession de ces étapes.

Selon encore un autre mode particulier de l'invention, l'huile siccative de l'invention est une huile de lin modifiée qui a subi deux étapes parmi celles considérées précédemment.

Selon un autre mode de réalisation particulier de l'invention, l'huile siccative est une huile de lin soufflée.

Le soufflage d'une huile se caractérise notamment par une polymérisation de ladite huile avec l'oxygène de l'air. L'huile soufflée peut être en particulier obtenue en soufflant de l'air au travers de l'huile chauffée.

Selon un mode particulier de réalisation, les compositions cosmétiques selon l'invention peuvent comprendre de 5 à 95 % en poids, notamment de 5 à 40 % en poids, en particulier de 5 à 15 % en poids d'huile siccative par rapport au poids total de la composition.

### Sels métalliques

Un vernis à ongles conforme à l'invention comprend au moins un sel métallique, et de préférence au moins deux sels métalliques distincts.

L'association d'au moins une huile siccative avec un(des) sel(s) métallique(s) permet notamment lors du séchage de former un film par réticulation avec l'oxygène de l'air.

Le(s) sels métallique(s) accélère(ent) en particulier le séchage des huiles siccatives naturelles.

Avantageusement, le sel métallique utilisé comprend moins de 5 % de cobalt par rapport au poids total du métal dans les sels métalliques, voire est avantageusement exempt de cobalt, en particulier afin d'obtenir un profil toxicologique plus favorable.

Selon un mode de réalisation particulier, le(s) sel(s) métallique(s) conforme(s) à l'invention est(sont) choisi(s) parmi les sels de manganèse, de calcium, de zirconium, de zinc, de strontium, de lithium, de cérium et de vanadium.

Selon un mode de réalisation particulier, le sel métallique est un sel organique. Il peut notamment présenter la formule (I) suivante : 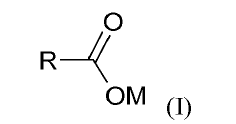 dans laquelle :
M est choisi parmi le manganèse, le calcium, le zirconium, le zinc, le strontium, le lithium, le cérium et le vanadium ; et
R est un radical (C₁-C₂₅)alkyle, linéaire ou ramifié, pouvant éventuellement comporter 1 à 3 insaturations.

Selon un mode particulier, R est un radical (C₆-C₂₀)alkyle, linéaire ou ramifié, pouvant éventuellement comporter 1 à 2 insaturations.

Selon un mode particulier, le sel métallique peut prendre la forme d'octoate, de linoléate, d'octanoate, d'oléate, de stéarate, de laurate ou de naphténate.

Selon un mode de réalisation particulier, un vernis à ongles conforme à l'invention comprend au moins un sel de manganèse.

Selon un autre mode de réalisation particulier de l'invention, la composition de vernis à ongles selon l'invention comprend plusieurs sels métalliques, en particulier dans le but d'augmenter la vitesse de réticulation de l'huile siccative.

Selon un mode de réalisation particulier de l'invention, les compositions cosmétiques de vernis à ongles selon l'invention comprennent de 0,001 à 2 % en poids, notamment de 0,01 à 1 % en poids, en particulier de 0,1 à 0,5 % en poids de métal par rapport à l'extrait sec.

Des exemples de produits commerciaux de sels métalliques sont donnés ci-après : Octa-Soligen Manganese 6HS, Octa-Soligen Zirconium 12 HS, et Octa-Soligen Calcium 5 HS, commercialisés par la société OMG BORCHERS GMBH.

L'indication xHS (6HS, 12HS et 5HS) employée ci-dessus, indique que le produit commercial ou matière première contient x pourcent en métal par rapport au poids total du produit commercial ou sel métallique.

Selon un mode particulier de l'invention, un vernis à ongles selon l'invention peut comprendre au moins deux sels métalliques différents choisis parmi les sels tels que définis précédemment.

Selon un autre mode particulier de l'invention, les sels métalliques d'un vernis à ongles de l'invention peuvent comprendre au moins un sel métallique primaire et au moins un sel métallique secondaire, voire peuvent consister en un sel métallique primaire et un sel métallique secondaire.

Dans le cadre de ce mode de réalisation particulier,
- le sel métallique primaire peut être choisi parmi les sels de manganèse, les sels de cérium et les sels de vanadium, et plus particulièrement, est un sel de manganèse ; et
- le sel métallique secondaire peut être choisi parmi les sels de zirconium, de zinc, de lithium et de strontium, et plus particulièrement, est un sel de zirconium.

Dans le cadre de ce mode de réalisation particulier, les sels métalliques d'un vernis à ongles de l'invention peuvent comprendre au moins un sel de manganèse et au moins un sel de zirconium.

Selon encore un autre mode de réalisation préféré de l'invention, un vernis à ongles de l'invention peut comprendre au moins un sel métallique primaire tel que défini précédemment, au moins un sel métallique secondaire tel que défini précédemment, et au moins un sel métallique auxiliaire.

Dans le cadre de ce dernier mode de réalisation particulier, le sel métallique auxiliaire peut être choisi parmi les sels de calcium.

Selon un mode de réalisation particulièrement préféré, les sels métalliques d'un vernis à ongles de l'invention peuvent comprendre au moins un sel de manganèse, au moins un sel de zirconium et au moins un sel de calcium, voire peuvent consister en un sel de manganèse, un sel de zirconium et un sel de calcium.

Dans le cadre de ce mode de réalisation particulièrement préféré, le sel de manganèse peut notamment favoriser le séchage superficiel et le séchage complet du film ; le sel de zirconium peut favoriser le séchage en profondeur, induisant un séchage régulier du film ; et le sel de calcium peut permettre de développer un effet synergétique entre le sel manganèse et le sel de zirconium.

Toujours selon ce mode de réalisation particulièrement préféré, la composition de vernis à ongles de l'invention peut comprendre au moins un sel métallique primaire tel que défini précédemment dans une teneur allant de 0,005 à 0,1 % en poids de métal par rapport à l'extrait sec, notamment de 0,01 à 0,05 % en poids de métal par rapport à l'extrait sec. Elle peut en outre comprendre au moins un sel métallique secondaire tel que défini précédemment dans une teneur allant de 0,005 à 0,8 % en poids de métal par rapport à l'extrait sec, notamment de 0,03 à 0,5 % en poids de métal par rapport à l'extrait sec. Elle peut enfin de plus comprendre au moins un sel métallique auxiliaire tel que défini précédemment dans une teneur allant de 0,03 à 0,3 % en poids de métal par rapport à l'extrait sec.

### Caractérisation de l'extrait sec

La teneur en matière sèche, communément appelée « extrait sec », c'est à dire la teneur en matière non volatile, peut être mesurée de différentes manières. On peut citer par exemple les méthodes par séchage à l'étuve, les méthodes par séchage par exposition à un rayonnement infrarouge.

De préférence, la quantité de matière sèche des compositions de vernis à ongles selon l'invention est mesurée par échauffement de l'échantillon par des rayons infrarouges de 2 µm à 3,5 µm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :
On étale environ 1g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.
La teneur en matière sèche est calculée de la manière suivante :
   Extrait Sec = 100 x (masse sèche / masse humide).
Les compositions de vernis à ongles selon l'invention se caractérisent par une teneur en matière sèche comprise entre 10 et 60 % et plus particulièrement entre 20 et 40 % en poids.

### Polymère filmogène

Un vernis à ongles selon l'invention comprend au moins un polymère filmogène.

Par « polymère filmogène », on désigne au sens de la présente invention, un polymère apte à former à lui seul ou en présence d'un agent auxiliaire de filmification, un film isolable, notamment continu et adhérent, sur un support, notamment sur les ongles.

En particulier, un polymère filmogène selon l'invention est distinct des huiles siccatives telles que décrites précédemment.

Dans la composition, on peut utiliser un seul polymère filmogène ou un mélange de polymères filmogènes.

Ce polymère filmogène peut être choisi dans le groupe constitué par les polymères synthétiques, de type radicalaire ou de type polycondensat, les polymères d'origine naturelle et leurs mélanges.

Un polymère filmogène convenant à l'invention peut être choisi en particulier parmi :
- les dérivés de polysaccharides, tels que les dérivés de cellulose ou de gomme de guar. Un dérivé de polysaccharides convenant à l'invention peut être un ester ou alkyléther de polysaccharide.

Par « ester ou alkyléther de polysaccharide », on désigne un polysaccharide formé de motifs répétitifs comportant au moins deux cycles identiques ou différents et présentant un degré de substitution par unité de saccharide compris entre 1,9 et 3 de préférence compris entre 2,2 et 2,9, et plus particulièrement entre 2,4 et 2,8. On désigne par substitution la fonctionnalisation des groupements hydroxyles en fonctions esters et/ou alkyléthers, et/ou la fonctionnalisation des groupements carboxyliques en fonctions esters.

Autrement dit, il peut s'agir d'un polysaccharide, partiellement ou totalement substitué par des groupements esters et/ou alkyléthers. De préférence, les groupements hydroxyles peuvent être substitués par des fonctions ester et/ou alkyléther en C₂-C₄.

On peut citer en particulier les esters de cellulose, tels que l'acétate de cellulose, les acétobutyrates de cellulose ou les acétopropianates de cellulose ; les alkyléthers de cellulose comme les éthylcelluloses, et les éthylguars ;
- les polymères synthétiques tels que les polyuréthanes, les polymères acryliques, les polymères vinyliques, les polyvinylbutyrals, les résines alkydes et les résines cétone/aldéhyde, les résines issues des produits de condensation d'aldéhydes, telles que les résines arylsulfonamide formaldéhyde comme la résine toluène sulfonamide formaldéhyde, les résines aryl-sulfonamide époxy ou encore les résines éthyl tosylamide ;
- les polymères d'origine naturelle, tels que les résines végétales telles que les dammars, l'élémi, les copals, le benjoin ; les gommes telles que la gomme laque, la gomme sandaraque et la gomme mastic.

Comme polymère filmogène, on peut notamment utiliser les résines toluène sulfonamide formaldéhyde « Ketjentflex MS80 » de la société AKZO ou « Santolite MHP », « Santolite MS 80 » de la société FACONNIER ou « RESIMPOL 80 » de la société PAN AMERICANA, la résine alkyde « BECKOSOL ODE 230-70-E » de la société DAINIPPON, la résine acrylique « ACRYLOID B66 » de la société ROHM & HAAS, la résine polyuréthane « TRIXENE PR 4127 » de la société BAXENDEN, la résine acétophénone/formaldéhyde commercialisée sous la référence Synthetic Resin SK par Degussa.

Selon un mode de réalisation particulier, le polymère filmogène est choisi parmi les polysaccharides et les dérivés de polysaccharide, de préférence parmi les éthers et les esters de polysaccharides, notamment en C₂-C₄, et plus préférentiellement est choisi parmi les acétobutyrates de cellulose, les acétopropianates de cellulose, l'acétate de cellulose, les éthylcelluloses, les éthylguars et leurs mélanges.

Selon un mode de réalisation particulièrement préféré, le polymère filmogène est choisi parmi l'éthylguar et l'éthylcellulose.

Selon un mode de réalisation particulier, un vernis à ongles de l'invention comprend une teneur totale en polymère filmogène compris entre 1 et 60 % en poids, notamment entre 2 et 30 % en poids, en particulier entre 5 et 15 % en poids, par rapport au poids total du vernis à ongles.

Selon un mode de réalisation préféré, une composition de vernis à ongles selon l'invention comprend moins de 5 % en poids en matière sèche de nitrocellulose, par rapport au poids total de la composition, de préférence moins de 3 % en poids, de préférence moins de 1,5 % poids, de préférence moins de 1 % en poids.

Selon un mode de réalisation particulièrement préféré, la composition de vernis à ongles de l'invention est totalement dépourvue de nitrocellulose.

Selon un mode particulier de l'invention, un vernis à ongles selon l'invention comprend au moins de l'éthylguar, au moins de l'huile de lin soufflée et au moins des sels de manganèse, de zirconium et de calcium.

### Milieu physiologiquement acceptable

La composition de l'invention comprend en outre un milieu physiologiquement acceptable. On désigne ainsi un milieu non toxique et notamment susceptible d'être appliqué sur les ongles.

Ce milieu peut être de type solvant organique ou de type dispersion d'une phase organique dans une phase continue aqueuse.

Le milieu physiologiquement acceptable de la composition comprend généralement au moins un solvant volatil. Ce solvant volatil peut être notamment choisi parmi les solvants organiques volatils, l'eau et leurs mélanges.

### Milieu solvant organique

La composition de vernis à ongle selon l'invention peut comprendre au moins un milieu solvant organique comprenant un ou plusieurs composés non aqueux liquides à température ambiante, appelés aussi huiles ou solvants organiques.

Le solvant organique peut être choisi parmi :
- les cétones liquides à température ambiante telles que la méthyléthylcétone, la méthylisobutylcétone, la diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyle, l'acétate de n-butyle, l'acétate d'isopentyle, l'acétate de méthoxypropyle, l'acétate de t-butyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tels que le carbonate de propylène, le carbonate de diméthyle ,
- les acétals tels que le méthylal ;
- et leurs mélanges.

Le milieu organique peut comprendre au moins une huile siliconée volatile.

Par « huile volatile », on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatils, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Les huiles siliconées volatiles, peuvent être linéaires ou cycliques volatiles, notamment celles ayant une viscosité ≤ 8 centistokes (8.10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I) : 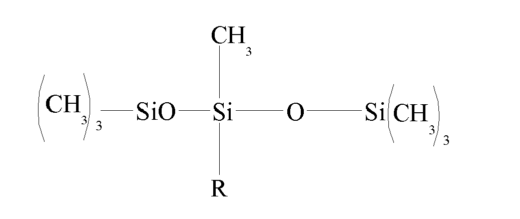 où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane, correspondant aux huiles de formule (I) pour lesquelles R est respectivement un groupe butyle, un groupe propyle ou un groupe éthyle.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyle, l'acétate de n-butyle, l'acétate d'isopentyle, l'acétate de méthoxypropyle, le lactate de butyle et leurs mélanges.

Le milieu solvant organique peut être de préférence l'acétate d'éthyle ou l'éthanol.

Le milieu solvant organique peut représenter de 10 à 95 % en poids, de préférence de 15 à 80 % en poids, et mieux de 20 à 70 % en poids par rapport au poids total de la composition.

### Milieu aqueux

La composition selon l'invention peut également comprendre au moins un milieu aqueux, constituant une phase aqueuse, qui peut former une phase continue dans laquelle est dispersée une phase organique, telle que décrite précédemment.

La phase aqueuse peut être constituée essentiellement d'eau ; elle peut également comprendre un mélange d'eau et de solvant(s) organique(s) miscible(s) à l'eau (miscibilité dans l'eau supérieure à 50 % en poids à 25 °C) comme les mono-alcools inférieurs ayant de 1 à 5 atomes de carbone tels que l'éthanol, l'isopropanol, les glycols ayant de 2 à 8 atomes de carbone, tels que le propylène glycol, l'éthylène glycol, le 1,3-butylène glycol, le dipropylène glycol, les cétones en C₃-C₄, les aldéhydes en C₂-C₄.

La phase aqueuse continue (eau et éventuellement solvant organique miscible à l'eau) peut être présente en une proportion variant de 30 à 90 % en poids, de préférence de 50 à 80 % en poids par rapport au poids total de la composition.

Selon un autre mode de réalisation, la composition comprend un milieu solvant organique continu et moins de 10 % en poids d'eau, de préférence moins de 5 % en poids d'eau par rapport au poids total de la composition.

### Agent auxiliaire de filmification

Pour améliorer les propriétés filmogènes de la composition de vernis à ongles un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, des tartrates, des phosphates, des sébaçates ;
- des dérivés oxyéthylénés, tels que les huiles oxyéthylénées, notamment des huiles végétales, telles que l'huile de ricin ;
- les huiles d'origine naturelle, en particulier non siccatives, choisies parmi des huiles comprenant au moins un acide gras choisi parmi l'acide caprylique, l'acide caprique, l'acide laurique, l'acide myristique, l'acide palmitique, l'acide stéarique, l'acide oléique, l'acide ricinoléique, l'acide linoléique, l'acide linolénique, l'acide arachidique, l'acide gadoléique, l'acide béhénique, l'acide érucique, l'acide brassidique, l'acide cétoléique, l'acide lignocérique, l'acide nervonique. En particulier, ces huiles sont choisies parmi les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées. Ces huiles sont notamment des triglycérides héptanoïques ou octanoïques, les huiles d'arachide, de babassu, de noix de coco, de pépins de raisin, de coton, de maïs, de germes de maïs, de graines de moutarde, de palme, de colza, de sésame, de soja, de tournesol, de germe de blé, de canola, d'abricot, de mangue, de ricin, de karité, d'avocat, d'olive, d'amande douce, d'amande, de pêche, de noix, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat, de beurre de karité ou encore les triglycérides des acides caprylique/caprique, et
- leurs mélanges.

Le type et la quantité d'agent plastifiant et/ou de coalescence peuvent être choisis par l'homme du métier sur la base de ses connaissances générales.

Par exemple, la teneur en agent(s) plastifiant(s) et/ou de coalescence peut aller de 0,01 % à 20 % et en particulier de 0,5 % à 10 % en poids par rapport au poids total de la composition.

### Agent gélifiant

La composition de vernis à ongles selon l'invention peut comprendre en outre un agent gélifiant.

Cet agent gélifiant peut en particulier être choisi parmi : les silices hydrophobes, telles que celles décrites dans le document EP-A-0 898 960, et par exemple commercialisées sous les références « AEROSIL R812^{®} » par la société Degussa, « CAB-O-SIL TS-530^{®} », « CAB-O-SIL TS-610^{®} », « CAB-O-SIL TS-720^{®} » par la société Cabot, « AEROSIL R972^{®} », « AEROSIL R974^{®} » par la société Degussa ; les argiles telles que la montmorillonite, les argiles modifiées telles que les bentones par exemple, l'hectorite stéaralkonium, la bentonite stéaralkonium, les alkyléther de polysaccharides (notamment dont le groupe alkyle comporte de 1 à 24 atomes de carbone, de préférence de 1 à 10, mieux de 1 à 6, et plus spécialement de 1 à 3) tels que ceux décrits dans le document EP-A-0 898 958.

La proportion totale en agent(s) gélifiant(s) dans les compositions selon l'invention peut aller de à 0,01 à 15 % en poids, par rapport au poids total de la composition, de préférence de 0,5 à 15 % et mieux de 0,5 à 10 % en poids.

### Matière colorante

La composition de vernis à ongles selon l'invention peut, en outre, comprendre une ou des matières colorantes choisies parmi les colorants solubles, et les matières colorantes pulvérulentes comme les pigments, les nacres, et les paillettes bien connues de l'homme du métier.

Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, polyester, polyuréthane, polyéthylène téréphtalate, les céramiques, les alumines et recouvert ou non de substances métalliques comme l'aluminium, l'or, le cuivre, le bronze, ou d'oxydes métalliques comme le dioxyde de titane, l'oxyde de fer, l'oxyde de chrome, de pigments minéraux ou organiques et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

On peut aussi utiliser des pigments à propriétés goniochromatiques, notamment à cristaux liquides ou multicouche.

Les colorants sont, par exemple, le rouge Soudan, le DC Red 17, le DC Green 6, le β-carotène, l'huile de soja, le brun Soudan, le DC Yellow 11, le DC Violet 2, le DC Orange 5, le jaune quinoléine.

La matière colorante peut également être choisie parmi les azurants optiques.

La composition peut comprendre, en outre des fibres éventuellement enrobées d'azurants optiques.

### Charge

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc.). On peut citer le talc, le mica, la silice, le kaolin, les poudres de polyamide (Nylon^{®}) (Orgasol^{®} de chez Atochem), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la société Dow Corning) et les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Additifs

La composition peut comprendre, en outre, d'autres ingrédients utilisés couramment dans les compositions cosmétiques et connus de l'homme du métier comme étant susceptibles d'être incorporés dans une composition de vernis à ongles.

De tels ingrédients peuvent être choisis parmi les huiles, les cires, les agents antiradicaux libres, les agents d'étalement, les agents mouillants, les agents dispersants, les anti-mousses, les conservateurs, les tensioactifs, les parfums, les neutralisants, les stabilisants, les antioxydants, les actifs pouvant être choisis parmi les huiles essentielles, les filtres UV/solaires, les agents hydratants, les vitamines, les protéines, les céramides, les extraits végétaux, etc. ; et leurs mélanges.

Les compositions selon l'invention peuvent comprendre en outre à titre d'actifs, des agents de soin des ongles tels que des agents durcissants pour matières kératiniques, des actifs agissant sur la croissance de l'ongle comme par exemple le méthyl sulfonyle méthane et/ou des actifs pour traiter des affections diverses localisées au niveau de l'ongle, comme par exemple l'onichomycose.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

### II - ARTICLE SOUPLE

Comme précisé précédemment, la présente invention concerne, selon un autre de ses aspects, un article souple destiné à être appliqué sur les ongles et/ou faux ongles pour leur maquillage et/ou leur soin, comprenant au moins une couche polymérique, **caractérisé en ce que** ladite couche polymérique comprend au moins une huile siccative, au moins un polymère filmogène et au moins un sel métallique.

D'une manière générale, l'article souple conforme à la présente invention se présente sous la forme d'un film ou laminé.

Au sens de la présente invention, le terme « souple » qualifie une flexibilité suffisante de ce film, c'est-à-dire propice à des déformations mécaniques de type étirement pour l'ajuster à la surface d'un ongle.

Par ailleurs, il faut également comprendre que par « souple », on entend apte à se déformer non élastiquement de manière à se conformer au profil plus ou moins bombé de l'ongle.

Cette déformabilité est notamment caractérisée par le paramètre de déformation à la rupture εᵣ discuté ci-après. L'article selon l'invention se différencie notamment à ce titre d'un article de type faux ongle qui se caractérise par une rigidité incompatible avec une telle déformation mécanique.

Une autre différence entre l'article conforme à l'invention et un faux ongle, réside dans la sensibilité de cet article vis-à-vis des solvants organiques polaires du type acétone, ester et/ou alcool court, tels que les acétates d'alkyle, notamment l'acétate d'éthyle.

En effet, l'article selon l'invention peut être aisément éliminé par démaquillage à l'aide d'un dissolvant classique ou solvant, par opposition à un faux ongle qui se retire. Ainsi, l'article selon l'invention est avantageusement démaquillable par des solvants organiques et notamment par l'acétone, et les acétates d'alkyles tel que l'acétate d'éthyle, et leurs mélanges.

L'invention concerne aussi un article susceptible d'être démaquillé à l'aide d'un solvant choisi parmi l'acétone, les acétates d'alkyles tel que l'acétate d'éthyle, et leurs mélanges.

A tous ces égards, l'article souple selon l'invention se distingue d'une part des compositions liquides classiques type vernis à ongle en raison de sa structure partiellement sèche, et d'autre part des produits solides types faux-ongles, par le fait que ledit article avant application est déformable mécaniquement sans la présence nécessaire de solvant résiduel, et peut se démaquiller, contrairement à un faux-ongle qui est apposé directement sur l'ongle puis limé pour être ajusté et ensuite directement enlevé de l'ongle afin de retrouver un ongle à l'état normal.

L'article selon l'invention peut être utilisé à des fins de maquillage, auquel cas il comprend des matières colorantes, ou à des fins de protection vis-à-vis d'un film de vernis. Dans cette alternative, la couche polymérique est généralement transparente.

Selon un mode de réalisation, l'article souple selon la présente invention présente une épaisseur allant de 6 µm à 1 mm, en particulier de 10 µm à 500 µm, et encore plus particulièrement de 50 µm à 200 µm.

L'épaisseur visée s'entend comme étant l'épaisseur avant application sur l'ongle de l'intégralité de la structure indissociable à une ou plusieurs couches comprenant notamment la couche polymérique.

En particulier, si la couche polymérique n'est pas auto-adhésive, et nécessite sur une de ses faces la présence d'une couche adhésive, l'épaisseur visée s'entend de l'épaisseur de l'ensemble des couches polymériques et adhésives.

En revanche, toute structure fixée de manière amovible à l'article souple, en particulier une pellicule de protection sur l'une ou l'autre des faces de l'article, en particulier, une pellicule siliconée sur la face adhésive de l'article, n'entre pas en ligne de compte dans la mesure de l'épaisseur.

L'invention a aussi pour objet un article souple destiné à être appliqué sur les ongles et/ou faux ongles pour leur maquillage et/ou leur soin tel que défini ci-dessus comprenant au moins une couche polymérique, **caractérisé en ce que** la dite couche polymérique comprend au moins une huile siccative, au moins un polymère filmogène et au moins un sel métallique, et présentant une première face adhésive comportant un matériau adhésif et destinée à être mise au contact de l'ongle, et une seconde face opposée à la première.

En particulier, l'article souple peut comporter en outre une pellicule de protection au contact de la première face de la couche polymérique, à enlever préalablement à la mise en place de l'article sur l'ongle. De préférence la face de la pellicule de protection au contact de la première face de la feuille est recouverte d'un matériau anti-adhésif, notamment siliconé.

Selon un mode de réalisation particulier, l'article conforme à l'invention est revêtu sur ses deux faces avec une pellicule amovible, identique ou différente.

L'article souple, et en particulier l'excès, peut être prédécoupé ou découpé, avant ou après son application, selon la taille et la forme désirée avec de petits ciseaux, un coupe ongles ou en grattant le film.

Le produit de maquillage selon l'invention est avantageusement conditionné, de préférence immédiatement après fabrication, dans un réservoir comme par exemple une poche, souple ou non, suffisamment étanche pour lui préserver cet aspect non totalement réticulé. Ce n'est qu'au moment de son utilisation et par conséquent lors de sa mise en contact avec l'air, que le produit réticule progressivement pour acquérir la dureté nécessaire à une bonne tenue.

Selon un mode particulier de l'invention, la couche polymérique est fabriquée par enduction d'une composition selon l'invention comprenant un solvant, de préférence l'acétone, les acétates d'alkyles tel que l'acétate d'éthyle, et leurs mélanges. L'enduction est séchée à l'abri de l'oxygène, de préférence sous azote, de manière à permettre l'évaporation du solvant sans faire réticuler l'huile siccative.

L'article souple ou article souple à l'état sec selon invention se présente notamment sous la forme d'un film non liquide qui peut se caractériser par un extrait sec élevé. En effet, la quantité de matière sèche dans cet article à l'état sec, à savoir une fois appliqué sur l'ongle ou le faux ongles, est supérieure ou égale à 95 % en poids par rapport au poids total de l'article. Autrement dit, la quantité de solvant volatil est inférieure ou égale à 5 % en poids par rapport au poids total de l'article.

Le produit de maquillage, selon un autre aspect évoqué précédemment comporte un article sous forme partiellement sèche. Dans ce cas aussi, le produit de maquillage est avantageusement conditionné dans un réservoir comme par exemple une poche, souple ou non, suffisamment étanche pour lui préserver cet aspect partiellement sec. Ce n'est qu'au moment de son utilisation et par conséquent lors de sa mise en contact avec l'air, que le produit sèche totalement pour acquérir la teneur en matière sèche décrite précédemment, conforme à l'article conforme à l'invention.

Dans un produit selon l'invention, l'article partiellement sec possède avantageusement une teneur en matière sèche supérieure à 80 %, notamment supérieure à 85 % et inférieure à 95 % en poids par rapport à son poids total.

Un tel article, lorsqu'il est extrait du conditionnement d'un produit conforme à l'invention et exposé à l'air ambiant, acquiert un état sec tel que défini ci-dessus au terme de 24 h.

De préférence, la quantité de matière sèche, ou encore extrait sec, des articles selon l'invention, est mesurée selon la méthode décrite précédemment pour la détermination de l'extrait sec d'une composition de l'invention.

Le protocole de mesure est le suivant : on dépose environ 10 g d'échantillon d'un article sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :
Extrait Sec = 100 x (masse sèche/ masse humide).

### Reprise à l'eau

L'article selon l'invention peut être avantageusement caractérisé à l'état sec par une reprise à l'eau portée à 25 °C inférieure ou égale à 20 %, notamment inférieure ou égale à 16 %, et en particulier inférieure à 10 %.

Selon la présente demande, on entend par « reprise à l'eau », le pourcentage d'eau absorbé par l'article après 60 minutes d'immersion dans l'eau, à 25 °C (température ambiante). La reprise à l'eau est mesurée pour des morceaux d'environ 1 cm découpés dans l'article sec. Ils sont pesés (mesure de la masse Ml) puis immergés dans l'eau pendant 60 minutes ; immersion, le morceau de film est essuyé pour éliminer l'excédent d'eau en surface puis pesé (mesure de la masse M2). La différence M2 - M1 correspond à la quantité d'eau absorbée par le film.

La reprise à l'eau est égale à [(M2 - Ml) / Ml] x 100 et est exprimée en pourcentage de poids par rapport au poids du film.

### Module de conservation E'

Par ailleurs, l'article selon l'invention est avantageusement un film ayant un module de conservation E' supérieur ou égal à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

La mesure du module de conservation est effectuée par DMTA (« *Dynamical and Mechanical Temperature Analysis* » ou « Analyse dynamique et mécanique en température »).

On effectue des essais de viscoélasticimétrie avec un appareil DMTA de Polymer TA Instruments (modèle DMA2980), sur un échantillon d'article. On découpe (par exemple à l'emporte-pièce) les éprouvettes. Celles-ci ont typiquement une épaisseur d'environ 150µm, une largeur de 5 à 10 mm et une longueur utile d'environ 10 à 15 mm.

Les mesures sont effectuées à une température constante de 30 °C.

L'échantillon est sollicité en traction et en petites déformations (on lui impose par exemple un déplacement sinusoïdal de ± 8 µm) lors d'un balayage en fréquence, la fréquence allant de 0,1 à 20 Hz. On travaille ainsi dans le domaine linéaire, sous de faibles niveaux de déformation.

Ces mesures permettent de déterminer le module complexe E* = E' + iE" du film de composition testé, E' étant le module de conservation et E" le module dit de perte.

### Déformation et/ou Energie à la rupture

Avantageusement, les articles selon l'invention, possèdent une déformation à la rupture εᵣ supérieure ou égale à 5 %, notamment allant de 5 à 500 %, de préférence supérieure ou égale à 15 %, notamment allant de 15 à 400 % et/ou une énergie à rupture par unité de volume Wr supérieure ou égale à 0,2 J/cm³, notamment allant de 0,2 à 100 J/cm³, de préférence supérieure à 1 J/cm³, notamment allant de 1 à 50 J/cm³.

La déformation à la rupture et l'énergie à rupture par unité de volume sont déterminées par des essais de traction effectués sur un film réticulé d'environ 200 µm d'épaisseur.

Pour effectuer ces essais, l'article est découpé en éprouvettes haltères de longueur utile 33 ±1 mm et de largeur utile 6 mm.

La section (S) de l'éprouvette est alors définie comme :

S = largeur x épaisseur (cm²) ; cette section sera utilisée pour le calcul de la contrainte.

Les essais sont réalisés, par exemple, sur un appareil de traction commercialisé sous l'appellation Lloyd^{®} LR5K. Les mesures sont réalisées à température ambiante (20 °C).

Les éprouvettes sont étirées à une vitesse de déplacement de 33 mm/min, correspondant à une vitesse de 100 % d'allongement par minute.

On impose donc une vitesse de déplacement et on mesure simultanément l'allongement ΔL de l'éprouvette et la force F nécessaire pour imposer cet allongement.

C'est à partir de ces données ΔL et F que l'on détermine les paramètres contraintes σ et déformation ε. Il est ainsi obtenu une courbe contrainte σ = (F/S) en fonction de la déformation ε = (ΔL/Lo) x 100, l'essai étant conduit jusqu'à rupture de l'éprouvette, Lo étant la longueur initiale de l'éprouvette. La déformation à la rupture εᵣ est la déformation maximale de l'échantillon avant le point de rupture (en pourcentage). L'énergie à rupture par unité de volume Wr en J/cm³ est définie comme la surface sous cette courbe contrainte/déformation telle que :

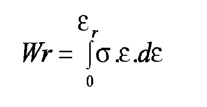

De préférence, l'article souple conforme à l'invention comprend plus de 80 % en poids de composés d'origine naturelle, de préférence plus de 90 %, de préférence plus de 95 %, de préférence ledit laminé comprend exclusivement des composés d'origine naturelle.

### COUCHE POLYMERIQUE

L'(les) huile(s) siccative(s), le(s) polymère(s) filmogène(s) et/ou le(s) sel(s) métallique(s) de la couche polymérique sont en particulier tels que décrits précédemment dans le cadre du vernis à ongles sous forme liquide ou gélifiée.

Concernant l'huile siccative, dans le cas de l'article souple, l'huile siccative ne comporte pas de groupements époxydes. Elle est qualifiée d'« huile siccative sans groupement époxyde » ou « huile non époxydée ».

Selon un mode particulier de réalisation, la couche polymérique de l'article souple selon l'invention comprend de 1 à 99 % en poids, notamment de 10 à 90 % en poids, en particulier de 30 à 70 % en poids d'huile siccative par rapport au poids total de la couche polymérique.

De préférence, la couche polymérique comprend outre de l'huile siccative et du polymère filmogène, au moins un sel métallique dans le but d'augmenter la vitesse de réticulation de l'huile siccative.

Concernant le polymère filmogène, dans le cas de l'article souple et selon un mode de réalisation particulier, le polymère filmogène est choisi parmi un éther ou un ester de cellulose. Selon un mode de réalisation particulièrement préféré, le polymère filmogène choisi est l'éthylcellulose.

La présente invention vise en particulier un article conforme à l'invention, **caractérisé en ce que** le polymère filmogène est choisi parmi les polysaccharides ou les dérivés de polysaccharide, de préférence un alkyléther ou un ester de cellulose, de préférence un acétobutyrate de cellulose, un acétopropionate de cellulose, l'éthylcellulose ou l'éthylguar.

Selon un mode particulier de réalisation, la couche polymérique de l'article souple selon l'invention comprend de 1 à 99 % en poids, notamment de 10 à 90 % en poids, en particulier de 30 à 70 % en poids de polymère filmogène en matière sèche par rapport au poids total de la couche polymérique.

Selon un mode particulier de réalisation, l'article selon l'invention comprend de 0,001 à 2 % en poids, notamment de 0,01 à 1 % en poids, en particulier de 0,1 à 0,5 % en poids de métal par rapport au poids total de l'extrait sec de la couche polymérique.

Toujours dans le cadre de la mise en oeuvre de l'article souple, de préférence, la couche polymérique conforme à l'invention comprend plus de 80 % en poids, de préférence plus de 90 % en poids, de préférence plus de 95 % en poids, de préférence la couche polymérique comprend exclusivement des composés d'origine naturelle par rapport au poids total de la couche polymérique.

A titre de composés additionnels éventuels dans la couche polymérique, on peut citer les agents auxiliaires de filmification. La couche polymérique peut comprendre en outre un agent auxiliaire de filmification qui peut être choisi parmi tous les composés connus de l'homme du métier comme étant susceptibles de remplir la fonction recherchée, et être notamment choisi parmi les agents plastifiants et les agents de coalescence du polymère filmogène.

En particulier, on peut citer, seuls ou en mélange, les composés éventuellement modifiés d'origine naturelle tels que les huiles comprenant au moins un acide gras telles que décrites ci-dessus dans le cadre de l'alternative du vernis à ongles sous forme liquide ou gélifiée.

Selon un mode particulier de réalisation, les couches adhésives selon l'invention comprennent de 0,1 à 70 % en poids, notamment de 5 à 60 % en poids, et plus particulièrement de 10 à 50 % en poids de plastifiant par rapport au poids total de l'extrait sec de la couche adhésive.

Selon un mode de réalisation de l'invention, l'article souple comprend un ester d'acide caprylique et/ou caprique ou un ester d'alcool caprylique et/ou caprique à titre de plastifiant, ledit ester étant d'origine végétale tel que défini ci-après.

On peut citer aussi les plastifiants ou agents de coalescence usuels, tels que: les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, les esters de glycol, les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther, les esters d'acides notamment carboxyliques tels que les citrates, notamment le citrate de triéthyle, le citrate de tributyle, l'acétylcitrate de triéthyle, l'acétylcitrate de tributyle, l'acétylcitrate de triéthyl-2 hexyle ; les phtalates, notamment le phtalate de diéthyle, le phtalate de dibutyle, le phtalate de dioctyle, le phtalate de dipentyle, le phtalate de diméthoxyéthyle ; les phosphates, notamment le phosphate de tricrésyle, le phosphate de tributyle, le phosphate de triphényle, le phosphate de tributoxyéthyle ; les tartrates, notamment le tartrate de dibutyle ; les adipates ; les carbonates ; les sébaçates ; le benzoate de benzyle, l'acétylricinoléate de butyle, l'acétylricinoléate de glycéryle, le glycolate de butyle, le camphre, le triacétate de glycérol, le N-éthyl-o,p-toluènesulfonamide, les dérivés oxyéthylénés tels que les huiles oxyéthylénées, les huiles de silicone, et leurs mélanges.

Le type et la quantité d'agent plastifiant et/ou de coalescence peuvent être choisis par l'homme du métier sur la base de ses connaissances générales. Par exemple, la teneur en agent plastifiant et/ou de coalescence peut aller de 0,01 % à 20 % et en particulier de 0,5 % à 10 % en poids par rapport au poids total de la couche polymérique.

### COUCHE ADHESIVE

En sus de la couche polymérique, l'article selon l'invention peut posséder une face externe adhésive si la couche polymérique n'est pas auto-adhésive. Une telle face adhésive est obtenue de façon générale grâce à la présence d'au moins une couche adhésive, **caractérisée en ce que** cette couche adhésive comprend au moins un matériau adhésif.

Par « matériau », on entend au sens de la présente invention un polymère ou un système polymérique pouvant comprendre un ou plusieurs polymères de natures différentes. Ce matériau adhésif peut en outre contenir un plastifiant.

Ce dernier doit présenter un certain pouvoir collant défini par ses propriétés viscoélastiques.

Les propriétés viscoélastiques d'un matériau sont classiquement définies par deux valeurs caractéristiques qui sont les suivantes :
- le module élastique qui représente le comportement élastique du matériau pour une fréquence donnée et qui est classiquement noté G',
- le module visqueux qui représente le comportement visqueux du matériau pour une fréquence donnée et qui est classiquement noté G".

Ces grandeurs sont notamment définies dans le « Handbook of Pressure Sensitive Adhesive Technology », 3d edition, D. Satas, chapter 9, pages 155 à 157.

De préférence, les matériaux adhésifs utilisables selon la présente invention présentent des propriétés viscoélastiques qui sont mesurées à une température de référence de 35 °C et dans un certain intervalle de fréquences.

Dans le cas de matériaux adhésifs sous forme de solution ou de dispersion de polymère dans un solvant volatil (tel que l'eau, un ester court, un alcool court, de l'acétone, etc), on mesure les propriétés viscoélastiques de ce matériau dans des conditions
dans lesquelles il présente une teneur en solvant volatil inférieure à 30 %, et, en particulier, une teneur en solvant volatil inférieure à 20 %.

On mesure, en particulier, le module élastique du matériau à trois fréquences différentes :
- à faible fréquence, soit à 2.10⁻² Hz ;
- à une fréquence intermédiaire, soit à 0,2 Hz ;
- à haute fréquence, soit à 2 Hz ; et
- le module visqueux à la fréquence de 0,2 Hz.

Ces mesures permettent d'évaluer l'évolution du pouvoir collant du matériau adhésif au cours du temps.

Ces propriétés viscoélastiques sont mesurées lors d'essais dynamiques sous sollicitations sinusoïdales de faible amplitude (petites déformations) réalisés à 35 °C sur une plage de fréquence allant de 2.10⁻² à 20 Hz sur un rhéomètre de type « Haake RS50^{®} »sous une sollicitation de torsion/cisaillement, par exemple en géométrie cône-plan (par exemple avec un angle du cône de 1°).

Avantageusement, ledit matériau adhésif répond aux conditions suivantes :
- G'(2 Hz, 35 °C) ≥ 10³ Pa,
- G'(35 °C) ≤ 10⁸ Pa, en particulier, G'(35 °C) ≤ 10⁷ Pa, et
- G' (2.10⁻² Hz, 35 °C) ≤ 3.10⁵ Pa,
   dans lesquelles :
   - G'(2 Hz, 35 °C) est le module élastique de cisaillement dudit matériau adhésif, mesuré à la fréquence de 2 Hz et à la température de 35 °C,
   - G'(35 °C) est le module élastique de cisaillement dudit matériau adhésif, mesuré à la température de 35 °C, pour toute fréquence comprise entre 2. 10⁻² et 2 Hz,
   - G'(2.10 Hz, 35 °C) est le module élastique de cisaillement dudit matériau adhésif, mesuré à la fréquence de 2.10⁻² Hz et à la température de 35 °C.

Dans une forme particulière de l'invention, le matériau adhésif répond également à la condition suivante :
- G"/G' (0,2 Hz, 35 °C) ≥ 0,35.
   dans laquelle :
   - G"(0,2 Hz, 35 °C) est le module visqueux de cisaillement dudit matériau adhésif, mesuré à la fréquence de 0,2 Hz et à la température de 35 °C,
   - G'(0,2 Hz, 35 °C) est le module élastique de cisaillement dudit matériau adhésif, mesuré à la fréquence de 0,2 Hz et à la température de 35 °C.

Dans une forme particulière de l'invention, on a :
- G'(2 Hz, 35 °C) ≥ 5.10³ Pa, et en particulier, G'(2 Hz, 35 °C) ≥ 10⁴ Pa.

Dans une autre forme particulière de l'invention, on a :
- G'(2.10⁻² Hz, 35 °C) ≤ 5.10⁴ Pa.

En particulier, les matériaux adhésifs selon l'invention répondent aux quatre conditions suivantes :
- G'(2 Hz, 35 °C) ≥ 10⁴ Pa, et
- G'(35 °C) ≤ 10⁸ Pa, en particulier G'(35 °C) ≤ 10⁷ Pa,
- G'(2.10⁻² Hz, 35 °C) ≤ 5.10⁴ Pa, et
- G"/G'(0,2 Hz, 35 °C) ≥ 0,35.

Les matériaux adhésifs peuvent être choisis parmi les adhésifs de type « *Pressure Sensitive Adhesives* » (adhésifs sensibles à la pression) par exemple, comme ceux cités dans le « Handbook of Pressure Sensitive Adhesive Technology », 3d edition, D. Satas.

Les matériaux adhésifs peuvent en particulier être des polymères choisis parmi:
- les polyuréthannes,
- les polymères acryliques,
- les gommes butyliques, notamment parmi les polyisobutylènes,
- les polymères éthylène-acétate de vinyle,
- les polyamides éventuellement modifiés par des chaînes grasses,
- les adhésifs siliconés, notamment les copolymères de résine de silicone et de silicone fluide, tels que ceux commercialisés par Dow Corning sous la référence Bio-PSA,
- et leurs mélanges.

Il peut en particulier s'agir de copolymères adhésifs dérivant de la copolymérisation de monomères vinyliques avec des entités polymériques, comme par exemple, ceux décrits dans le brevet US 6,136,296. Sont également susceptibles de convenir à l'invention, les copolymères adhésifs décrits dans le brevet US 5,929,173 possédant un squelette polymérique, de Tg variant de 0 °C à 45 °C, greffés par des chaînes dérivant de monomères acryliques et/ou méthacryliques et possédant en revanche une Tg variant de 50 °C à 200 °C.

Ces matériaux adhésifs peuvent également être des polymères choisis parmi les copolymères blocs ou statistiques comprenant au moins un monomère ou une association de monomères dont le polymère résultant à une température de transition vitreuse inférieure à la température ambiante (25 °C), ces monomères ou associations de monomères pouvant être choisis parmi le butadiène, l'éthylène, le propylène, l'isoprène, l'isobutylène et leurs mélanges. Des exemples de tels matériaux sont les polymères blocs de type styrène-butadiène-styrène, styrène-(éthylène-butylène)-styrène, styrène-isoprène-styrène comme ceux vendus sous les dénominations commerciales « Kraton^{®} » de Shell Chemical Co. ou de «Vector^{®} » de la société Dexco Polymers.

Les matériaux adhésifs sont par exemple choisis parmi les polyisobutylènes présentant une masse molaire relative Mv supérieure ou égale à 10 000 et inférieure ou égale à 150 000. En particulier, cette masse molaire relative est supérieure ou égale à 18 000 et inférieure ou égale à 150 000.

Comme produits commerciaux convenant particulièrement bien à la présente invention, on peut citer les polyisobutylènes de masses molaires relatives Mv respectives 40 000, 55 000 et 85 000 vendus sous les dénominations commerciales respectives « Oppanol B 10^{®} », « Oppanol B 12^{®} » et « Oppanol B 15^{®} » par la société BASF, et leurs mélanges.

Selon un mode de réalisation particulier, le matériau adhésif comprend au moins un caoutchouc naturel, au moins une résine tackifiante et éventuellement au moins un plastifiant.

Au sens de l'invention, on entend par caoutchouc naturel, un caoutchouc que l'on obtient à partir de végétaux, et qui peut optionnellement avoir été modifié notamment par réaction chimique. De préférence, le caoutchouc naturel ne subit aucune modification chimique après extraction.

Selon un mode privilégié de l'invention, le caoutchouc naturel est obtenu après élimination de la phase aqueuse d'un latex naturel de polyisoprène, choisi de préférence parmi les latex d'hévéa, de Parthernium Argentatum (guayule), de solidago virgaurea minuta, de Taraxacum koksaghyz, de Gutta percha, de Gutta balata, de sapotillier (Manilkara zapota) et leurs mélanges.

De préférence, les latex naturels sont déprotéinisés, en particulier pour réduire les risques d'allergies.

A titre de résines tackifiantes, on peut citer les colophanes ou dérivés de colophane tels que les colophanes hydrogénées, les esters de colophane, les esters de colophane hydrogénés, les terpènes, les résines hydrocarbonnées aliphatiques ou aromatiques, les résines phénoliques, les résines styréniques et coumarone-indène. Sont considérées aussi comme résines tackifiantes la gomme de laque, la gomme sandaraque, le dammar, l'élémi, les copals, le benjoin, la gomme mastic ou leurs mélanges. Ces dernières sont particulièrement avantageuses dans le cadre de la présente invention.

### Plastifiant

Les matériaux adhésifs pouvant être mis en oeuvre dans la couche de la présente invention peuvent en outre comporter des plastifiants, de préférence naturels ou d'origine naturelle

Les plastifiants conformes à l'invention sont de préférences choisis parmi les composés éventuellement modifiés d'origine naturelle tels que les huiles comprenant au moins un acide gras choisi parmi l'acide caprylique, l'acide caprique, l'acide laurique, l'acide myristique, l'acide palmitique, l'acide stéarique, l'acide oléique, l'acide rinoléique, l'acide linoléique, l'acide linolénique, l'acide arachidique, l'acide gadoléique, l'acide béhénique, l'acide érucique, l'acide brassidique, l'acide cétoléique, l'acide lignocérique, l'acide nervonique et un mélange de ceux-ci, et en particulier les huiles comprenant un mélange d'acide caprylique et caprique.

En particulier, les huiles convenant à la mise en oeuvre de l'invention sont choisies parmi les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées. Ces huiles sont notamment des triglycérides héptanoïques ou octanoïques, les huiles d'arachide, de babassu, de noix de coco, de pépins de raisin, de coton, de maïs, de germes de maïs, de graines de moutarde, de palme, de colza, de sésame, de soja, de tournesol, de germes de blé, de canola, d'abricot, de mangue, de ricin, de karité, d'avocat, d'olive, d'amande douce, d'amande de pêche, de noix, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat, de beurre de karité ou encore les triglycérides des acides caprylique/caprique, et leurs mélanges.

Selon un mode particulier de réalisation, les couches adhésives selon l'invention comprennent de 0,1 à 70 % en poids, notamment de 5 à 60 % en poids, et plus particulièrement de 10 à 50 % en poids de plastifiant par rapport au poids total de l'extrait sec de la couche adhésive.

Selon un mode de réalisation de l'invention, l'article souple comprend un ester d'acide caprylique et/ou caprique ou un ester d'alcool caprylique et/ou caprique à titre de plastifiant, ledit ester étant d'origine végétale.

On entend par acide ou alcool caprylique et/ou caprique des acides carboxyliques ou alcools linéaires ou ramifiés, de préférence linéaires, ayant 8 ou 10 carbones.

Au sens de l'invention, on entend par « ester d'origine végétale », un ester que l'on obtient à partir de matières premières végétales ou d'origine végétale.

Cet ester peut répondre à l'une des formules (I) à (X) suivantes :

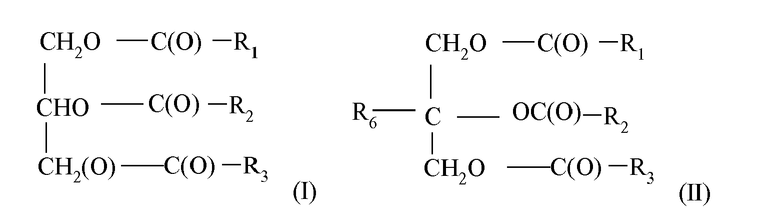

R₁-C(O)-OCH₂-C(CH₃)₂CH₂O-C(O)-R₂ (III)

R₁-C(O)-O(Y)_{d}-C(O)-R₂ (IV)

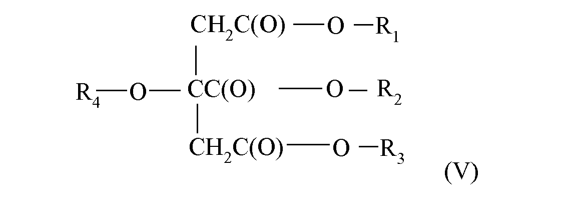

R₇-O-C(O)-R₁ (VI)

CH₃-(CH₂)₅-CH(CH₃)O-C(O)-R₁ (VII)

R₆-O-C(O)-R₁ (VIII)

R₇-O-C(O)-R₆ (IX)

R₇-O-C(O)-(CH₂)₈-C(O)-O-R₈ (X)

dans lesquelles :
R₁, R₂, R₃, identiques ou différents, représentent l'atome d'hydrogène ou une chaîne alkyle saturée, linéaire ou ramifiée, de préférence linéaire, à 7 ou 9 atomes de carbone,
R₄ représente l'atome d'hydrogène ou un groupement (Alk)-C(CO)-, dans lequel (Alk) représente une chaîne alkyle linéaire ou ramifiée comprenant entre 1 et 5 atomes de carbone,
R₆ représente une chaîne alkyle linéaire ou ramifiée, substituée ou non, comprenant entre 1 et 18 atomes de carbone, en particulier entre 1 et 10, ou une chaine alcényle linéaire ou ramifiée, substituée ou non, comprenant entre 2 et 18 atomes de carbone, en particulier entre 2 et 10,
Y est un groupe ethoxy, isopropoxy ou propoxy,
d est compris entre 1 et 1000, en particulier compris entre 1 et 15, en particulier égal à 1, et
R₇, R₈, identiques ou différents, représentent une chaîne alkyle saturée, linéaire ou ramifiée, de préférence linéaire, à 8 ou 10 atomes de carbone,
L'ester conforme à l'invention peut également comprendre un mélange de différents esters définis ci-dessus.

De tels esters sont commercialement disponibles, par exemple auprès de la société Cognis ou RTD Hallstar.

Selon un mode de réalisation préféré de l'invention, ce plastifiant peut notamment être un composé de formule (I) tel que défini ci-dessus, ou autrement dit un triglycéride d'acide caprylique et/ou caprique.

Selon un autre mode de réalisation préféré de l'invention, ce plastifiant peut notamment être un composé de formule (VII) tel que défini ci-dessus, ou autrement dit un caprylate et/ou caprate d'isooctyle.

Selon un mode particulier de l'invention, la couche adhésive est composée d'au moins un caoutchouc naturel, éventuellement déprotéinisé, tel qu'un latex naturel de polyisoprène dépourvu de sa phase aqueuse, d'au moins une résine tackifiante parmi celles mentionnées ci-dessus, de préférence d'au moins une résine tackifiante naturelle ou d'origine naturelle, et d'au moins un plastifiant, de préférence naturel ou d'origine naturelle, parmi ceux mentionnés ci-dessus.

La couche adhésive dans l'article conforme à l'invention est généralement sous la forme d'une couche ayant une épaisseur de 5 à 100 µm, en particulier de 10 à 80 µm, de préférence de 15 à 50 µm, de préférence de 20 à 30 µm.

D'une manière générale, l'adhésif est tel que ledit article ne peut être ôté par pelage lorsqu'il est appliqué à la surface d'un ongle synthétique ou naturel après au moins 24 heures de pose.

De préférence, la couche adhésive conforme à l'invention comprend plus de 80 % en poids, de préférence plus de 90 % en poids, de préférence plus de 95 % en poids, de préférence la couche adhésive comprend exclusivement des composés d'origine naturelle par rapport au poids total de la couche adhésive.

### Solvant d'origine végétale

L'article selon l'invention peut comprendre un milieu solvant constitué d'un ou plusieurs solvants d'origine végétale. Autrement dit, selon ce mode de réalisation, la composition est alors complètement exempte de solvants d'origine non végétale.

Au sens de l'invention, on entend par « solvant d'origine végétale », un solvant que l'on obtient directement à partir de végétaux et n'ayant subi aucune modification chimique après extraction.

Les solvants d'origine végétale conformes à l'invention sont de préférence choisis parmi les alcools et/ou les acétates et/ou les lactates.

Selon un mode de réalisation préféré, le solvant d'origine végétale est notamment choisi parmi l'éthanol et/ou l'acétate d'éthyle et/ou le lactate d'éthyle.

De préférence, il s'agira d'un mélange d'éthanol et d'acétate d'éthyle.

L'éthanol exclusivement d'origine végétale est largement disponible auprès de nombreux fournisseurs, notamment auprès de la société TEREOS. L'acétate d'éthyle d'origine végétale est disponible auprès de la société Rhodia. Le lactate d'éthyle est disponible auprès de la société REACT-NTI sous la référence ENVIROSOLVE SILVER.

Selon un mode particulier de réalisation, l'article souple ou article souple à l'état sec selon l'invention comprend de 1 à 20 % de milieu solvant en poids, notamment de 1 à 10 % en poids, en particulier de 1 à 5 % en poids par rapport au poids total de l'article souple.

### Autres additifs

### i) Pigments et colorants

La couche adhésive selon l'invention peut comprendre notamment au moins une matière colorante, organique ou inorganique, notamment de type pigments ou nacres classiquement utilisée dans les vernis sous forme liquide ou gélifiée, tels que décrits précédemment.

Les pigments peuvent être présents à raison de 0,01 à 20 % en poids, notamment de 0,01 à 15 % en poids, et en particulier de 0,02 à 10 % en poids, par rapport au poids total de l'article souple.

### ii) Matériau à effet

La couche adhésive et la couche polymérique selon l'invention peuvent contenir au moins un matériau à effet optique spécifique. Cet effet est différent d'un simple effet de teinte conventionnel, c'est-à-dire unifié et stabilisé tel que produit par les matières colorantes classiques décrite plus haut comme par exemple les pigments monochromatiques. Au sens de l'invention, « stabilisé » signifie dénué d'effet de variabilité de la couleur avec l'angle d'observation ou encore en réponse à un changement de température.

Ce matériau est présent en quantité suffisante pour produire un effet optique perceptible à l'oeil nu. Avantageusement, il s'agit d'un effet choisi parmi les effets goniochromatique, métallique et notamment miroir, *soft-focus*, arc en ciel et/ou thermochrome et/ou photochrome.

Par exemple, ce matériau peut être choisi parmi les particules à reflet métallique, les agents de coloration goniochromatiques, les pigments diffractants, les agents thermochromes, photochromiques les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

### Particules à reflet métallique

Par « particules à reflet métallique », on désigne des particules dont la nature, la taille, la structure et l'état de surface leur permettent de réfléchir la lumière incidente notamment de façon non iridescente.

Les particules présentant une surface extérieure sensiblement plane conviennent également, car elles peuvent plus facilement donner naissance, si leur taille, leur structure et leur état de surface le permettent, à une réflexion spéculaire intense que l'on peut alors qualifier d'effet miroir.

Les particules à reflet métallique utilisables dans l'invention, peuvent par exemple réfléchir la lumière dans toutes les composantes du visible sans absorber de manière significative une ou plusieurs longueurs d'ondes. La réflectance spectrale de ces particules peut par exemple être supérieure à 70 % dans l'intervalle 400-700 nm, et mieux d'au moins 80 %, voire 90 % ou encore 95 %.

Ces particules ont généralement une épaisseur inférieure ou égale à 1 µm, notamment inférieure ou égale à 0,7 µm, en particulier inférieure ou égale à 0,5 µm

La proportion totale en particules à reflet métallique est notamment inférieure ou égale à 20 % en poids et en particulier inférieure ou égale à 10 % en poids par rapport au poids total de la première composition ou du film organique et/ou minéral.

Les particules à reflet métallique utilisables dans l'invention sont en particulier choisies parmi:
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures.

Parmi les dérivés métalliques pouvant être présents dans lesdites particules, on peut citer notamment les oxydes métalliques tels que par exemple les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

Selon une première variante, les particules à reflet métallique peuvent être composées d'au moins un métal tel que défini précédemment, d'au moins un dérivé métallique tel que défini précédemment, ou bien encore d'un de leurs mélanges.

Ces particules peuvent être au moins partiellement recouvertes par une couche d'un autre matériau, par un exemple de matériau transparent tel que notamment du colophane, de la silice, des stéarates, des polysiloxanes, des résines polyesters, des résines époxydiques, des résines polyuréthanes et des résines acryliques.

A titre illustratif de ces particules, on peut citer des particules d'aluminium, telles que celles commercialisées sous les dénominations « STARBRITE 1200 EAC^{®}» par la société Siberline et METALURE^{®} par la société Eckart.

On peut également citer les poudres métalliques de cuivre ou des mélanges d'alliage telles les références 2844 commercialisées par la société Radium Bronze, les pigments métalliques comme l'aluminium ou le bronze, telles que celles commercialisées sous les dénominations « ROTOSAFE 700 » de la société Eckart, les particules d'aluminium enrobé de silice commercialisées sous la dénomination « VISIONAIRE BRIGHT SILVER » de la société Eckart et les particules d'alliage métallique comme des poudres de bronze (alliage cuivre et zinc) enrobé de silice commercialisées sous la dénomination de « VISIONAIRE BRIGHT NATURAL GOLD » de la société Eckart.

Selon une seconde variante, ces particules peuvent être des particules comportant un substrat et qui présentent donc une structure multicouche par exemple bicouche. Ce substrat peut être organique ou minéral, naturel ou synthétique, monomatière ou multimatériaux, plein ou creux. Lorsque le substrat est synthétique, il peut être réalisé avec une forme favorisant la formation d'une surface réfléchissante après revêtement, notamment après le dépôt d'une couche de matériaux à reflet métallique. Le substrat peut, par exemple, présenter une surface plane et la couche de matériaux à reflet métallique une épaisseur sensiblement uniforme.

Le substrat peut être en particulier choisi parmi les métaux et les dérivés métalliques tels que cités précédemment, et également parmi les verres, les céramiques, les alumines, les silices, les silicates et notamment aluminosilicates et les borosilicates, le mica synthétique tel que le fluorophlogopite, et leurs mélanges, cette liste n'étant pas limitative.

La couche à reflet métallique peut enrober en totalité ou en partie le substrat et cette couche peut être au moins partiellement recouverte par une couche d'un autre matériau, par exemple un matériau transparent notamment tel que cité précédemment. Selon un mode de réalisation particulier, cette couche à reflet métallique enrobe en totalité, directement ou indirectement, c'est-à-dire avec interposition d'au moins une couche intermédiaire métallique ou non, le substrat.

Les métaux ou dérivés métalliques pouvant être utilisés dans la couche réfléchissante sont tels que définis précédemment. Par exemple, elle peut être formée d'au moins un métal choisi parmi l'argent, l'aluminium, le chrome, le nickel, le molybdène, l'or, le cuivre, l'étain, le magnésium et leurs mélanges (alliages). On utilise plus particulièrement l'argent, le chrome, le nickel, le molybdène, et leurs mélanges.

A titre illustratif de ce second type de particules, on peut plus particulièrement citer :
Des particules de verre recouvertes d'une couche métallique notamment celles décrites dans les documents JP-A-09188830, JP-A-10158450, JP-A-10158541, JP-A-07258460 et JP-A-05017710.

A titre illustratif de ces particules comportant un substrat de verre, on peut citer celles revêtues respectivement d'argent, d'or ou de titane, en forme de plaquettes, commercialisées par la société Nippon Sheet Glass sous les dénominations « MICROGLASS METASHINE ». Des particules à substrat de verre revêtu d'argent, en forme de plaquettes, sont vendues sous la dénomination « MICROGLASS METASHINE REFSX 2025 PS » par la société Toyla. Des particules à substrat de verre revêtu d'alliage nickel/chrome/molybdène sont vendues sous la dénomination « CRYSTAL STAR GF 550, GF 2525 » par cette même société. Celles revêtues soit d'oxyde de fer brun, soit d'oxyde de titane, d'oxyde d'étain ou d'un de leur mélange comme celles commercialisées sous la dénomination « REFLECKS » par la société Engelhard ou celles commercialisées sous la référence « METASHINE MC 2080GP » par la société Nippon Sheet Glass.

Ces particules de verre recouvertes de métaux peuvent être enrobées de silice comme celles commercialisées sous la dénomination « METASHINE série PSS1 ou GPS1 » par la société Nippon Sheet Glass.

Des particules à substrat de verre sphérique revêtu ou non par un métal, notamment celles vendues sous la dénomination « PRIZMALITE MICROSPHERE » par la société Prizmalite Industries.

Conviennent également à l'invention, les pigments de la gamme « METASHINE 1080R » commercialisée par la société Nippon Sheet Glass CO Ltd. Ces pigments, plus particulièrement décrits dans la demande de brevet JP 2001-11340, sont des paillettes de verre C-GLASS comprenant 65 à 72 % de SiO₂, recouvertes d'une couche d'oxyde de titane de type rutile (TiO₂). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO₂.

Des particules comportant un substrat de borosilicate enrobé d'argent, encore appelées « nacres blanches ».

Des particules à substrat métallique tel que l'aluminium, le cuivre, le bronze, en forme de plaquettes, sont vendues sous la dénomination commerciale « STARBRITE » par la société Silberline et sous la dénomination « VISIONAIRE » par la société Eckart.

Des particules comportant un substrat de mica synthétique revêtu de dioxyde de titane, et par exemple les particules de dimension comprise entre 80 et 100 µm, comportant un substrat de mica synthétique (fluorophlogopite) revêtu de dioxyde de titane représentant 12 % du poids total de la particule, vendues sous la dénomination « PROMINENCE » par la société Nihon Koken.

Les particules à reflet métallique peuvent encore être choisies parmi les particules formées d'un empilement d'au moins deux couches à indices de réfraction différents. Ces couches peuvent être de nature polymérique ou métallique et notamment inclure au moins une couche polymérique.

Ainsi, les particules à effet métallique peuvent être des particules dérivant d'un film polymérique multicouche.

Le choix des matériaux destinés à constituer les différentes couches de la structure multicouche est bien entendu effectué de manière à conférer l'effet métallique souhaité aux particules ainsi formées.

De telles particules sont notamment décrites dans WO 99/36477, US 6,299,979 et US 6,387,498 et plus particulièrement identifiées ci-après dans le chapitre goniochromatique.

### Pigments diffractants

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinusoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Une structure possible pour le pigment diffractant peut comporter une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : MgF₂, SiO₂, Al₂O₃, AlF₃, CeF₃, LaF₃, NdF₃, SmF₂, BaF₂, CaF₂, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs composés, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : MgF₂, SiO, SiO₂, Al₂O₃, TiO₂, WO, AIN, BN, B4C, WC, TiC, TiN, N₄Si₃, ZnS, des particules de verre, des carbones de type diamant et leurs associations.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : MgF₂/Al/MgF₂, un pigment diffractant ayant cette structure étant commercialisé sous la dénomination « SPECTRAFLAIR 1400 Pigment Silver » par la société Flex Products, ou « SPECTRAFLAIR 1400 Pigment Silver FG ». La proportion en poids du MgF₂ peut être comprise entre 80 et 95 % du poids total du pigment.

### Agents de coloration goniochromatiques

Au sens de l'invention, un agent de coloration goniochromatique permet d'observer un changement de couleur, encore appelé « *color flop* », en fonction de l'angle d'observation, supérieur à celui que l'on peut rencontrer avec des nacres. On peut utiliser simultanément un ou plusieurs agents de coloration goniochromatique.

L'agent de coloration goniochromatique peut être choisi de manière à présenter un changement de couleur relativement important avec l'angle d'observation.

L'agent de coloration goniochromatique peut ainsi être choisi de telle sorte que l'on puisse observer, pour une variation de l'angle d'observation comprise entre 0° et 80° sous un éclairage à 45°, une variation de couleur ΔE de la composition cosmétique, mesurée dans l'espace colorimétrique CIE 1976, d'au moins 2.

L'agent de coloration goniochromatique peut également être choisi de telle sorte que l'on puisse observer, pour un éclairage à 45° et une variation de l'angle d'observation comprise entre 0° et 80°, une variation Dh de l'angle de teinte de la composition cosmétique, dans le plan CIE 1976, d'au moins 30°, voire au moins 40° ou au moins 60°, voire encore d'au moins 100°.

L'agent de coloration goniochromatique peut être choisi par exemple parmi les structures multicouches interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société Dupont de Nemours ; Cr/MgF₂/Al/MgF₂/Cr, des pigments ayant cette structure étant commercialisés sous la dénomination « CHROMAFLAIR » par la société Flex ; MoS₂/SiO₂/Al/SiO₂/MoS² , Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination « SICOPEARL » par la société BASF ; MoS₂/SiO₂/mica-oxyde/SiO₂/MoS₂ ; Fe₂0₃/SiO₂/mica-oxyde/SiO₂/Fe₂O₃ ; TiO₂/SiO₂/TiO₂ et TiO₂/Al₂O₃/TiO₂ SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination « XIRONA » par la société Merck (Darmstadt). A titre d'exemple, ces pigments peuvent être les pigments de structure silice/oxyde de titane/oxyde d'étain commercialisés sous le nom « XIRONA MAGIC » par la société MErck, les pigments de structure silice/oxyde de fer brun commercialisés sous le nom « XIRONA INDIAN SUMMER » par la société Merck et les pigments de structure silice/oxyde de titane/mica/oxyde d'étain commercialisés sous le nom « XIRONA CARRIBEAN BLUE » par la société Merck. On peut encore citer les pigments « INFINITE COLORS » de la société Shisheido. Selon l'épaisseur et la nature des différentes couches, on obtient différents effets. Ainsi, avec la structure Fe₂O₃/SiO₂/Al/ SiO₂/Fe₂O₃, on passe du doré-vert au gris-rouge pour des couches de SiO₂ de 320 à 350 nm ; du rouge au doré pour des couches de SiO₂ de 380 à 400 nm ; du violet au vert pour des couches de SiO2 de 410 à 420 nm ; du cuivre au rouge pour des couches de SiO₂ de 430 à 440 nm.

On peut encore utiliser des agents de coloration goniochromatique à structure multicouche comprenant une alternance de couches polymériques.

A titre illustratif, des matériaux pouvant constituer les différentes couches de la structure multicouche, on peut citer, cette liste n'étant pas limitative: le naphthalate de polyéthylène (PEN) et ses isomères par exemple 2,6-, 1,4-, 1,5-, 2,7- et 2,3-PEN, les téréphthalates de polyalkylene, des polyimides, des polyéthérimides, des polystyrènes atactiques, des polycarbonates, des polyméthacrylates et des polyacrylates d'alkyle, du polystyrène syndiotactique (sPS), des poly-alpha-méthylstyrène syndiotactiques, du polydichlorostyrène syndiotactique, copolymères et mélange de ses polystyrènes, des dérivés de cellulose, des polymères polyalkylènes, des polymères fluorés, des polymères chlorés, des polysulfones, des polyéthersulfones, des polyacrylonitriles, des polyamides, des résines siliconées, des résines époxy, de l'acétate de polyvinyle, des polyéthers-amides, des résines ionomériques, des élastomères et polyuréthanes. Conviennent également des copolymères, par exemple copolymères de PEN (par exemple, copolymères de 2,6-, 1,4-, 1,5-, 2,7-, et/ou 2,3-acide naphtalène dicarboxylique ou ses esters avec (a) acide téréphtalique ou ses esters ; (b) l'acide isophtalique ou ses esters ; (c) acide phtalique ou ses esters ; (d) des alcanes glycols ; (e) des cycloalkanes glycols (par exemple le cyclohexane diméthanol diol) ; (f) des acides alcanes dicarboxyliques ; et/ou (g) des acides cycloalkanes dicarboxylique, des copolymères de téréphthalates de polyalkylène et des copolymères de styrène. En outre, chaque couche individuelle peut inclure des mélanges de deux ou plusieurs polymères ou copolymères précédents. Le choix des matériaux destinés à constituer les différentes couches de la structure multicouche est bien entendu effectué de manière à conférer l'effet optique souhaité aux particules ainsi formées.

On peut citer, à titre d'exemple de pigments à structure multicouche polymérique, ceux commercialisés par la société 3M sous la dénomination « COLOR GLITTER ».

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société Chenix ainsi que celle commercialisées sous la dénomination « HELICONE^{®} HC » par la société Wacker.

Ces agents peuvent également être sous forme de fibres goniochromatiques dispersées. De telles fibres peuvent par exemple présenter une taille comprise entre 50 µm et 700 µm, par exemple d'environ 300 µm. En particulier, on peut utiliser des fibres interférentielles à structure multicouche. Des fibres à structure multicouche de polymères sont notamment décrites dans les documents EP-A-0 921 217, EP-A-0 686 858 et US-A-5,472,798. La structure multicouche peut comporter au moins deux couches, chaque couche, indépendamment ou non de la (ou les) autre(s) couche(s), étant réalisée en au moins un polymère de synthèse. Les polymères présents dans les fibres peuvent avoir un indice de réfraction allant de 1,30 à 1,82 et mieux allant de 1,35 à 1,75. Les polymères préférés pour constituer les fibres sont les polyesters tels que le polyéthylène téréphtalate, le polyéthylène naphtalate, le polycarbonate ; les polymères acryliques comme le polyméthacrylate de méthyle ; les polyamides.

Des fibres goniochromatiques à structure bicouche polyéthylène téréphtalate/nylon-6 sont commercialisées par la société Teijin sous la dénomination « MORPHOTEX ».

Dans une variante, cet agent de coloration goniochromatique peut être associé à au moins un pigment diffractant.

La combinaison de ces deux matériaux résulte en une composition ou un film qui présente une variabilité de la couleur accrue, donc qui est susceptible de permettre à un observateur de percevoir un changement de couleur, voire un mouvement de couleur, dans de nombreuses conditions d'observation et d'illumination.

Le rapport pondéral du pigment diffractant par rapport à l'agent de coloration goniochromatique est de préférence compris entre 85/15 et 15/85, mieux entre 80/20 et 20/80, mieux encore 60/40 et 40/60, par exemple de l'ordre de 50/50. Un tel rapport est favorable à l'obtention d'un effet arc-en-ciel et d'un effet goniochromatique soutenus.

### Azurants optiques

Les azurants optiques sont des composés bien connus de l'homme du métier.

De tels composés sont notamment décrits dans « Fluorescent Whitening Agent, Encyclopedia of Chemical Technology, Kirk-Othmer », vol. 11, pages 227-241, 4ème édition, 1994, Wiley. On peut plus particulièrement les définir comme des composés qui absorbent essentiellement dans l'UVA entre 300 et 390 nm et réémettent essentiellement entre 400 et 525 nm. Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et leurs mélanges. De tels composés sont facilement disponibles commercialement.

On peut citer par exemple : le dérivé stilbénique de naphto-triazole vendu sous la dénomination commerciale « Tinopal GS », le di-styryl-4,4' biphényle sulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC », le 4,4'-bis[(4,6-dianilino-1,3,5-triazin-2-yl)amino]stilbène-2,2'-disulfonate de sodium vendu sous la dénomination commerciale « Tinopal SOP », le 4,4'-bis-[(4-anilino-6-bis(2-dydroxyéthyl)amino-1,3,5-triazin-2-yl)amino]stilbène-2,2'-disulfonic acide vendu sous la dénomination commerciale « Tinopal UNPA-GX », le 4,4'-bis-[anilino-6-morpholine-1,3,5-triazin-2-yl)amino]stilbène vendu sous la dénomination commerciale « Tinopal AMS-GX », le 4,4'-bis-[(4-anilino-6-(2-hydroxyéthyl)méthylamino-1,3,5-triazin-2-yl)amino]stilbène-2,2'-disodium sulfonate vendu sous la dénomination commerciale « Tinopal 5BM-GX », tous par la société Ciba Spécialités Chimiques, le 2,5 thiophène di-yl bis (5 ter-butyl-1,3 benzoxazole) vendu sous la dénomination commerciale « Uvitex OB » par la société Ciba, le dérivé anionique du di-aminostilbène en dispersion dans l'eau vendu sous la dénomination commerciale « Leucophor BSB liquide » par la société Clariant, les laques d'azurant optique vendues sous la dénomination commerciale « COVAZUR » par la société Wackherr.

Les azurants optiques utilisables dans la présente invention peuvent aussi se présenter sous la forme de copolymères, par exemple d'acrylates et/ou de méthacrylates, greffés par des groupements azurants optiques comme décrits dans la demande FR 9 910 942.

Ils peuvent être utilisés tels quels ou introduits dans le film sous forme de particules et/ou de fibres recouvertes dudit azurant, telles que celles décrites ci-après.

En particulier, on peut utiliser les fibres recouvertes d'azurant optique telles que commercialisées par la société LCW sous la référence commerciale Fiberlon 54 ZO₃, ayant une longueur d'environ 0,4 mm et un titre de 0,5 deniers.

### Matériau à effet relief

L'effet relief peut ou non être associé à un effet optique. Un matériau de ce type est généralement présent en quantité suffisante pour conférer un effet relief perceptible au toucher voire à l'oeil nu. Il peut notamment s'agir d'un effet rugueux et/ou martelé.

### Matériau conférant un aspect rugueux

Les particules de forme sensiblement sphérique ou ovoïdale peuvent conférer un toucher doux au maquillage. Avantageusement, les particules solides ont une forme sensiblement sphérique, pour permettre leur bonne répartition lors de leur application.

Les particules solides utilisées selon l'invention peuvent avoir une taille moyenne allant de 2,5 µm à 5 mm, et mieux de 50 µm à 2 mm. Plus les particules sont de petites tailles, plus la tenue des particules est satisfaisante. L'emploi de particules est également compatible avec la réalisation de motifs.

Les particules solides peuvent être en tout matériau satisfaisant les propriétés de densités définies précédemment. Par exemple, les particules solides peuvent être en un matériau choisi parmi le verre, l'oxyde de zirconium, le carbure de tungstène, les plastiques comme les polyuréthanes, les polyamides, le polytétrafluoroéthylène, le polypropylène, les métaux comme l'acier, le cuivre, le laiton, le chrome, le marbre, l'onyx, le jade, la nacre naturelle, les pierres précieuses (diamant, émeraude, rubis, saphir), l'améthyste, l'aigue-marine. On utilise de préférence des billes de verres telles que celles vendues sous la dénomination « SILIBEADS^{®} » par la société Sigmund Lindner ; ces billes ont en outre pour avantage de conférer également un effet brillant et un scintillement au maquillage.

Les particules solides, déformables ou non, peuvent être pleines ou creuses, incolores ou colorées, enrobées ou non.

En ce qui concerne les fibres utilisables selon l'invention, il peut s'agir de fibres d'origine synthétique ou naturelle, minérale ou organique.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Il peut notamment s'agir de fibres utilisées dans la fabrication des textiles et notamment des fibres de soie, de coton, de laine, de lin, des fibres de cellulose, notamment extraite du bois, des légumes ou des algues, de rayonne, de polyamide (Nylon^{®}), de viscose, d'acétate notamment d'acétate de rayonne, de poly-(p-phénylène-téréphtalamide) (ou d'aramide) notamment de Kevlar^{®}, de polymère acrylique notamment de polyméthacrylate de méthyle ou de poly 2-hydroxyéthyl méthacrylate, de polyoléfine et notamment de polyéthylène ou de polypropylène, de verre, de silice, de carbone, notamment sous forme graphite, de polytétrafluoroéthylène (comme le Téflon^{®}), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

### Matériau conférant un aspect martelé

Les inventeurs ont également constaté qu'il était possible de conditionner dans l'invention un matériau comprenant un mélange silice pyrogéné, pigment métallique et composé organopolysiloxane pour lui conférer un aspect martelé.

Un tel mélange est notamment décrit dans la demande de brevet EP 1 040 813.

### iii) Matériau à effet olfactif

Avantageusement, l'article selon l'invention peut également être doté de propriétés olfactives par incorporation notamment dans l'une au moins des couches d'au moins un matériau odorant ou encore substance parfumante.

La substance parfumante peut être choisie parmi toute substance odoriférante bien connue de l'homme du métier, et notamment parmi les huiles essentielles et/ou les essences.

Ce matériau olfactif peut, si nécessaire, être introduit via un solvant-plastifiant.

On entend par « solvant-plastifiant » un composé qui solubilise au moins partiellement le matériau olfactif et qui est susceptible de s'évaporer lentement.

Le solvant-plastifiant peut être choisi parmi des glycols tels que le dipropylène glycol, l'éthyldiglycol, le n-propylglycol, le n-butylglycol, le méthyldiglycol, le n-butyldiglycol, des alcools tels que le cyclohexanol, l'éthyl-2 butanol, le méthoxy-3 butanol, l'éthyl-2 hexanol, le phénoxyéthanol, des esters, tels que le monoacétate de glycol, l'acétate d'éthylglycol, l'acétate de n-butylglycol, l'acétate d'éthyldiglycol, l'acétate de n-butyldiglycol, l'abiétate de méthyle, le myristate d'isopropyle, le diacétate de propylène glycol, l'acétate d'éther méthylique du propylène glycol, des éthers de glycols tels que l'éther méthylique du dipropylène glycol, l'éther butylique du dipropylène glycol, seuls ou en mélange.

L'une quelconque des couches de l'article selon l'invention peut également contenir un ou plusieurs additifs de formulation couramment utilisés en cosmétique et plus spécialement dans le domaine cosmétique et/ou soin des ongles. Ils peuvent notamment être choisis parmi les vitamines, les oligo-éléments, les adoucissants, les séquestrants, les agents alcalinisants ou acidifiants, les agents mouillants, les agents épaississants, les agents dispersants, les anti-mousses, les agents d'étalement, les co-résines, les conservateurs, les filtres UV, les actifs, les agents hydratants, les neutralisants, les stabilisants, les antioxydants et leurs mélanges.

Ainsi, ils peuvent notamment incorporer, à titre d'actifs, des agents durcissants ou renforçateur pour matières kératiniques, des actifs favorisant la pousse de l'ongle tel que le méthylsulfonylméthane et/ou des actifs pour traiter des affections diverses localisées au niveau de l'ongle, comme par exemple des actifs antimycotiques ou antimicrobiens.

Les quantités de ces différents ingrédients sont celles classiquement utilisées dans ce domaine et par exemple de 0,01 à 20 % en poids, et notamment de 0,01 à 10 % en poids par rapport au poids total de la feuille souple et/ou du matériau adhésif.

L'invention est illustrée plus en détail dans les exemples suivants qui sont présentés à titre illustratif et non limitatif de l'invention.

Sauf indication contraire, les quantités sont données en pourcentage en poids en matière première par rapport au poids total de la composition.

### EXEMPLES

### Exemple 1

| | |
|---|---|
| Ethylguar | 10 % |
| Huile de lin soufflée « LIN SOUFFLE CV 5P » ⁽¹⁾ | 10% |
| Octoate de manganèse ⁽²⁾ | 0,6 % |
| Octoate de zirconium ⁽³⁾ | 1,8 % |
| Octoate de calcium, neutre ⁽⁴⁾ | 1,6 % |
| Acétate d'éthyle | 76 % |

| | |
|---|---|
| ⁽¹⁾ commercialisé par la société Novance ⁽²⁾ Octa-Soligen Manganese 6HS commercialisé par la société OMG BORCHERS GMBH ⁽³⁾ Octa-Soligen Zirconium 12 HS commercialisé par la société OMG BORCHERS GMBH ⁽⁴⁾ Octa-Soligen Calcium 5 HS commercialisé par la société OMG BORCHERS GMBH | |

### Exemple référence (sans sel métallique)

| | |
|---|---|
| Ethylguar | 10 % |
| Acétate d'éthyle | 90 % |

### Mode opératoire :

L'éthylguar et l'huile de lin sont solubilisés dans l'acétate d'éthyle.

Les sels métalliques sont ensuite ajoutés.

Des films de ces deux compositions sont enduits à une épaisseur de 50 µm humide sur carte de contraste.

### Résultat :

Après 24 heures de séchage à 30 °C, la composition de l'exemple 1 forme un film solide, brillant, et adhérent sur carte de contraste.

La composition référence forme un film moins adhérent, que l'on parvient à décoller de la carte de contraste.

### Exemple 2

Dans cet exemple, une première composition (voir tableau suivant) est enduite à 300 µm humide sur un contre-adhésif (« Scotchpak 1022 Release liner 3.0mil » disponible chez 3M).

| | |
|---|---|
| Ethylcellulose ⁽¹⁾ | 10 % |
| Huile de lin ⁽²⁾ | 10 % |
| Acétate d'éthyle d'origine végétale ⁽³⁾ | 75 % |
| Octoate de Manganèse ⁽⁴⁾ | 0.6 % |
| Octoate de Zirconium ⁽⁵⁾ | 1.8 % |
| Octoate de Calcium ⁽⁶⁾ | 1.6 % |
| Pigments | 1.0 % |

| | |
|---|---|
| (1) Ethocel Standard 45 Premium, commercialisée par la société Dow Chemical. (2) Lin Technipol Supra, commercialisée par la société Novance. (3) Commercialisée par la société Rhodia. (4) Octa-Soligen Manganese 6HS, commercialisée par la société Borchers. (5) Octa-Soligen Zirconium 12 HS, commercialisée par la société Borchers. (6) Octa-Soligen Calcium 5 HS, neutre, commercialisée par la société Borchers. | |

Le film de la première composition est séché sous courant d'azote, de manière à permettre l'évaporation du solvant sans faire réticuler l'huile de lin. Le film de la première composition est ensuite mis en contact avec une couche adhésive de type PSA (Bio-PSA 7-4602, Dow Corning) préalablement enduite sur un contre-adhésif. Une pression est exercée pour faire adhérer les films entre eux. Le laminé obtenu est découpé pour avoir la forme d'ongles, puis est introduit dans un packaging sensiblement étanche pour éviter la poursuite de la réticulation de l'huile siccative. On obtient alors un produit de maquillage et/ou de soin des ongles et/ou faux ongles dans lequel est conditionné l'article souple partiellement sec.

Lors de l'application sur les ongles, le packaging est ouvert juste avant d'appliquer le laminé sur les ongles. On retire avec précaution les contre-adhésifs. Avant réticulation de l'huile de lin, l'huile de lin permet au laminé d'être étiré facilement lors de l'application sur les ongles. Progressivement dans le temps, la couche polymérique devient plus dure.

## Revendications

1. Vernis à ongles, **caractérisé en ce qu'**il comprend au moins une huile siccative, au moins un polymère filmogène et au moins un sel métallique et **en ce qu'**il comprend moins de 5 % en poids en matière sèche de nitrocellulose, par rapport au poids total du vernis à ongles.

2. Vernis à ongles, **caractérisé en ce qu'**il comprend au moins une huile siccative, au moins un polymère filmogène et au moins un sel métallique, ledit polymère filmogène étant choisi parmi les éthers et les esters de polysaccharides, notamment en C₂-C₄, et plus préférentiellement les acétobutyrates de cellulose, les acétopropianates de cellulose, l'acétate de cellulose, les éthylcelluloses, les éthylguars et leurs mélanges.

3. Vernis à ongles selon la revendication 1 ou 2, **caractérisé en ce que** l'huile siccative est choisie parmi l'huile de lin, l'huile de bois de Chine, l'huile d'oïticica, l'huile de vernonia, l'huile d'oeillette, l'huile de grenade, l'huile de calendula ; les esters de ces huiles végétales ; les résines alkydes obtenues à partir de ces huiles végétales, et leurs mélanges ; ladite huile siccative pouvant éventuellement être modifiée par réaction chimique.

4. Vernis à ongles selon l'une quelconque des revendications précédentes, **caractérisé en ce que** l'huile siccative est l'huile de lin raffinée ou soufflée.

5. Vernis à ongles selon l'une quelconque des revendications précédentes, **caractérisé en ce que** l'huile siccative est présente en une teneur allant de 5 à 95 % en poids, notamment de 5 à 40 % en poids, en particulier de 5 à 15 % en poids par rapport au poids total du vernis à ongles.

6. Vernis à ongles selon l'une quelconque des revendications précédentes, **caractérisé en ce que** le sel métallique comprend au moins un sel métallique choisi parmi les sels de manganèse, de calcium, de zirconium, de zinc, de strontium, de lithium, de cérium et de vanadium, et en particulier est un sel organique présentant la formule (I) suivante : 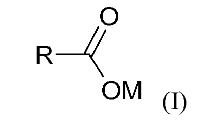 dans laquelle :
M est choisi parmi le manganèse, calcium, zirconium, zinc, strontium, lithium, cérium et vanadium ; et
R est un radical (C₁-C₂₅)alkyle, linéaire ou ramifié, pouvant éventuellement comporter 1 à 3 insaturations, et plus particulièrement un radical (C₆-C₂₀)alkyle, linéaire ou ramifié, pouvant éventuellement comporter 1 à 2 insaturations ;
et en particulier, prend la forme d'octoate, de linoléate, d'octanoate, d'oléate, de stéarate, de laurate ou de naphténate.

7. Vernis à ongles selon l'une quelconque des revendications précédentes, **caractérisé en ce qu'**il comprend au moins un sel de manganèse.

8. Vernis à ongles selon l'une quelconque des revendications précédentes, **caractérisé en ce que** le sel métallique comprend au moins deux sels métalliques différents choisis parmi les sels tels que définis en revendication 6, en particulier au moins un sel de manganèse et au moins un sel de zirconium, et plus particulièrement au moins un sel de manganèse, au moins un sel de zirconium et au moins un sel de calcium.

9. Vernis à ongles selon l'une quelconque des revendications précédentes, **caractérisé en ce que** le(s) sel(s) métallique(s) est(sont) présent(s) en une teneur allant de 0,001 à 2 % en poids, notamment de 0,01 à 1 % en poids et en particulier de 0,1 à 0,5 % en poids de métal par rapport à l'extrait sec.

10. Vernis à ongles selon l'une quelconque des revendications 1 et 3 à 9, **caractérisé en ce que** le polymère filmogène est choisi parmi les polysaccharides et les dérivés de polysaccharides, de préférence les éthers et les esters de polysaccharides, notamment en C₂-C₄, et plus préférentiellement les acétobutyrates de cellulose, les acétopropianates de cellulose, l'acétate de cellulose, les éthylcelluloses, les éthylguars et leurs mélanges.

11. Vernis à ongles selon l'une quelconque des revendications précédentes, **caractérisé en ce** le polymère filmogène est choisi parmi l'éthylguar et l'éthylcellulose.

12. Vernis à ongles selon l'une quelconque des revendications précédentes, **caractérisé en ce que** la teneur totale en polymère filmogène est comprise entre 1 et 60 % en poids, notamment entre 2 et 30 % en poids, et plus particulièrement entre 5 et 15 % en poids, par rapport au poids total du vernis à ongles.

13. Vernis à ongles selon l'une quelconque des revendications précédentes, **caractérisé en ce qu'**il comprend moins de 5 % en poids en matière sèche de nitrocellulose, par rapport au poids total du vernis à ongles, de préférence moins de 3 % en poids, de préférence moins de 1,5 % poids, de préférence moins de 1 % en poids, voire est totalement dépourvu de nitrocellulose.

14. Procédé cosmétique de maquillage et/ou de soin non thérapeutique des ongles comprenant l'application sur les ongles d'au moins une couche de vernis à ongles selon l'une quelconque des revendications précédentes.

15. Article souple destiné à être appliqué sur les ongles et/ou faux ongles pour leur maquillage et/ou leur soin, comprenant au moins une couche polymérique, **caractérisé en ce que** ladite couche polymérique comprend au moins une huile siccative, au moins un polymère filmogène et au moins un sel métallique.
